# EUROPEAN PATENT SPECIFICATION

(11) **EP 3 359 122 B1**
(45) Date of publication and mention of the grant of the patent: **26.06.2019**
(21) Application number: 17707029.9
(22) Date of filing: 24.02.2017
(51) Int. Cl.: A61K 8/58, A61Q 5/12, A61Q 19/00, A61K 8/891, A61K 8/893, A61K 8/894

(54) **USE OF ALKYL GLYCOSIDE MODIFIED POLYSILOXANES IN PURE OIL-BASED PERSONAL CARE PRODUCTS**
VERWENDUNG VON ALKYLGLYKOSID-MODIFIZIERTEN POLYSILOXANEN IN REINÖLBASIERTEN KÖRPERPFLEGEPRODUKTEN
UTILISATION DE POLYSILOXANES MODIFIÉS PAR DES ALKYLGLYCOSIDES DANS DES PRODUITS DE SOINS PERSONNELS À BASE D'HUILE PURE

(30) Priority: 29.02.2016 CN 201610107509
(43) Date of publication of application: 15.08.2018
(73) Proprietor: Wacker Chemie AG, 81737 München (DE)
(72) Inventor: WANG, Tracy, Zhenjiang Jiangsu 212004 (CN); XU, Eric, Shanghai 201615 (CN)
(74) Representative: Deffner-Lehner, Maria
(86) International application number: PCT/EP2017/054384
(87) International publication number: WO 2017/148827

(56) References cited:
- JP-A- H0 741 414
- US-A- 6 066 326
- US-A1- 2009 257 968
- DATABASE GNPD [Online] MINTEL; January 2015 (2015-01), "rose super damage repair serum", XP002769028, Database accession no. 2926889
- DATABASE GNPD [Online] MINTEL; October 2012 (2012-10), "Hair Multi Vitamin & Nutrients Capsules", XP002769029, Database accession no. 1896691
- wacker: "Silicone Polyglycosides : BELSIL WO 5000 and BELSIL SPG 128 VP", 2014 SCC Supplier's day , 2014, XP002769030, Retrieved from the Internet: URL:https://www.ulprospector.com/documents /1277029.pdf?bs=5139&b=215730&st=20&r=eu&i nd=personalcare [retrieved on 2017-04-06]
- Anonymous: "Belsil SPG 128 VP", Wacker Silicones , 1 December 2011 (2011-12-01), XP002769031, Retrieved from the Internet: URL:http://www.brenntag.com/media/document s/bsi/product_data_sheets/life_science/wac ker_silicones_pc/belsil_spg_128_vp_pds.pdf [retrieved on 2017-04-06]

## Description

### Field of the Invention

The invention relates to applications of alkyl glycoside modified polysiloxanes in pure oil-based personal care products.

### Background of the Invention

WO 03/075864 A1 discloses applications of a polyglycerol modified polysiloxane in oily cosmetics, claiming that it is free of stickiness, soft and smooth. The application examples in oily cosmetics include lip sticks, roll-on antiperspirants and oily liquid foundations. In oily liquid foundations therein, however, emulsifiers based on fatty acid esters are needed to help the powder suspend in the systems.

US6066326 discloses a cosmetic composition comprising at least one co-emulsifier and one polydimethylsiloxane containing glucoside groups. In Examples 1-4 disclosed therein, the polydimethylsiloxane containing glucoside groups is used in water-in-oil emulsions.

US6881416B2 discloses a process for the preparation of water-in-oil emulsions used as foundations by mixing WACKER BELSIL SPG 128 VP and WACKER BELSIL RG 100 with powders.

US5831080 describes a process for preparing a glycoside modified polysiloxane.

US6919071 discloses a water-in-oil sunscreen emulsion, where glycoside modified siloxane(s) is/are used as emulsifier(s).

WO2015091378A discloses a novel alkyl glycoside polysiloxane elastomer gel, whose crosslinked molecular chains contain silicone resin segments.

Both DATABASE GNPD [Online] MINTEL; January 2015 (2015-01), "rose super damage repair serum", XP002769028, Database accession no. 4206615 and DATABASE GNPD [Online] MINTEL; October 2012 (2012-10), "Hair Multi Vitamin & Nutrients Capsules", XP002769029, Database accession no. 1896691 disclose the use of caprylyl dimethicone glucoside in pure oil base hair care products.

In WACKER: "Silicone Polyglycosides: BELSIL® WO 5000 and BELSIL® SPG 128 VP", 2014 NY SCC Supplier's day, 2014, XP002769030 the trademarks BELSIL® WO 5000 and BELSIL® SPG 128 VP are described wherein BELSIL® WO 5000 consists of caprylyl dimethicone ethoxyglucoside in dimethicone and BELSIL® SPG 128 VP consists of caprylyl dimethicone ethoxyglucoside in cyclopentasiloxane and wherein both are suitable as personal care compositions.

WACKER: "BELSIL® SPG 128 VP", 1 December 2011 (2011-12-01), XP002769031 is the technical data sheet of BELSIL® SPG 128 VP and discloses solubility tests in various oils and fatty products.

In US 2009/257968 A1 oil based anhydrous compositions containing octyl dimethicone ethoxy glucoside are described which are suitable as personal care products.

Both JP H07 41414 A and US 6 066 326 A describe compositions based on alkyl glycoside modified polysiloxanes but JP H07 41414 A does not disclose longer alkyl groups bonded to the Si atom and US 6 066 326 A does not disclose pure-oil products.

### Summary of the Invention

The invention surprisingly discovers that alkyl glycoside modified polysiloxanes can be employed in pure oil-based personal care products to significantly reduce the stickiness of the pure oil-based products, in pure oil-based hair conditioner compositions to significantly improve the smoothness of hairs, and in pure oil-based skin care compositions and containing powders to significantly enhance the stability of compositions having a high powder content and to impart them with an excellent powder suspensibility.

The pure oil-based personal care products in the invention include pure oil-based hair conditioner compositions (including leave-on hair conditioner compositions), pure oil-based skin care compositions (including facial skin care compositions, particularly those having a high powder content).

"Pure oil-based" in this invention means that the pure oil-based products comprise less than or equal to 1 wt% of water, preferably less than or equal to 0.1 wt% of water. It could be a composition mainly containing oils ingredients and water content less than or equal to 1 wt%, preferably less than or equal to 0.1 wt% of water.

The leave-on hair conditioner compositions in the invention refer to the hair conditioner compositions that, after applied to the hairs, have a retention time greater than or equal to 1 hour. Preferably, "Leave-on" means that the hair is not rinsed until 8 hours after applying the hair conditioner.

The volatile hydrophobic solvents in the invention refer to hydrophobic solvents whose evaporation rate is greater than or equal to 0.1 wt%/min tested at 23°C according to DIN53249: 2007-05 and which are typically suitable for personal care products, including cyclopentasiloxane, isododecane and isohexadecane. "Hydrophobic" means that the solubility of the solvent in water is equal or less than 1 wt% at 25°C at 1013 hPa.

The nonvolatile oils in the invention refer to the oils whose evaporation rate is less than 0.1 wt%/min tested at 23°C according to DIN53249, including organosilicon oils such as dimethiconols (INCI name), which are α,ω-dihydroxy-terminated polydimethylsiloxanes, preferably with a viscosity of from 0.1 to 2,000 mPas at 25°C (according to DIN 53019), dimethicones (INCI name), which are dimethyl polysiloxanes, preferably with a viscosity of from 0.1 to 2,000 mPas at 25°C (according to DIN 53019), and phenyl modified dimethicones (INCI name), which are phenyl modified dimethyl polysiloxanes, preferably with a viscosity of from 0.1 to 2,000 mPas at 25°C (according to DIN 53019); hydrocarbon oils such as white spirit (a mixture of aliphatic and alicyclic C7 to C12 hydrocarbons), liquid paraffin, micro-crystalline wax, beeswax, C12-15 benzoate, palmitic acid, panthenol, cetanol, myristyl alcohol, isopropyl palmitate, sorbitan stearate, ceteareth-3, glyceryl laurates and ethylhexyl methoxycinnamate; vegetable oils such as sunflower oil, castor oil, jojoba oil, shea butter, avocado and grape seed oils; and animal fats such as lanolin and mink oils.

INCI names are described in the "International Cosmetic Ingredient Dictionary & Handbook", 16th Edition, from the Personal Care Product Council (ed.).

The powders for personal care products in the invention refer to inorganic and polymeric powders with nano-or micron-level particle size used to adjust colors or block UV in the field of personal care products, including silica, talc, mica, titanium dioxide, color pigment powders (such as iron oxide yellow, iron oxide black, iron oxide red, etc.), kaolin, zinc oxide and polymethacrylate.

The alkyl glycoside modified polysiloxanes in the invention can be prepared by the method as described in CN102492147B. The alkyl glycoside modified polysiloxanes in the invention are not crosslinked polymers and have a linear polysiloxane backbone with part of side groups being substituted by alkyl and glycoside groups.

The invention is as defined in the set of claims.

The invention provides for the use of alkyl glycoside modified polysiloxanes in pure oil-based personal care products, wherein the pure oil-based products comprise less than or equal to 1 wt% of water, preferably less than or equal to 0.1 wt% of water, and the structure of the alkyl glycoside modified polysiloxanes is as shown in Formula (I) 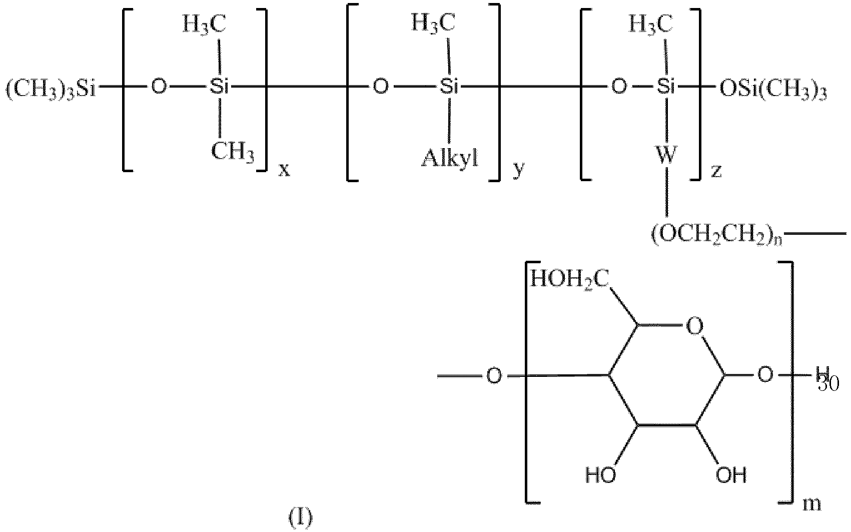
where x, y and z are the same or different and respectively represent positive integers between 1 and 10,000, preferably positive integers between 1 and 5,000;
n and m are the same or different and respectively represent positive integers between 1 and 20, preferably positive integers between 1 and 15;
Alkyl represents a linear or branched C₂-C₂₀ hydrocarbon chain; and preference is given to at least one alkyl group selected from a group of ethyl, propyl, butyl, pentyl, hexyl, heptyl and octyl;
W represents a linear or branched C₂-C₂₀ alkylene selected from a group of ethylene, propylene, butylene, pentylene, hexylene, heptylene and octylene.

According to the use as mentioned above, the alkyl glycoside modified polysiloxanes are used preferably in amounts of from 0.1 to 50 wt% in pure oil-based personal care products.

As in the application as mentioned above, the pure oil-based personal care products are preferably leave-on hair conditioner compositions having a turbidity of less than 80 NTU (**N**ephelometric **T**urbidity **U**nit), preferably less than 30 NTU, tested according to ISO 7027-1:2016 standard, measured preferably by the turbidimeter HACH-2100Q and its user instructions at room temperature.

In the use claim of the invention, the pure oil-based personal care products are hair conditioner compositions that contain:
(a) 0.1-50 wt% of alkyl glycoside modified polysiloxanes;
(b) 0-85 wt% of volatile hydrophobic solvents;
(c) 0-20 wt% of non-volatile oils;
(d) 0.1-10 wt% of dimethicone/vinyl dimethicone crosspolymer where the amount of components (b) and (c) cannot be zero at the same time and the percentage in each case is based on the total weight of the pure oil-based compositions.

Dimethicone/vinyl dimethicone crosspolymer (INCI name) is a crosslinked dimethyl siloxane polymer, preferably a silicone elastomer, formed by the reaction between a dimethyl polysiloxane having Si-bonded hydrogen and a dimethyl polysiloxane having Si-bonded vinyl groups, for example by the reaction of hydrogen dimethicone (INCI name; dimethyl polysiloxane having Si-bonded hydrogen) or bis-hydrogen dimethicone (INCI name; α,ω-dihydrogen dimethyl polysiloxane), and vinyl dimethicone (INCI name, dimethyl polysiloxane having Si-bonded vinyl groups) or bis-vinyldimethicone (INCI name, α,ω-divinyl dimethyl polysiloxane).

According to the use as mentioned above, the pure oil-based hair conditioner compositions preferably contain:
(a) 0.1-30 wt% of alkyl glycoside modified polysiloxanes;
(b) 20-85 wt% of volatile hydrophobic solvents;
(c) 5-35 wt% of non-volatile oils containing 0.5-35 wt% of dimethiconols;
where the percentage in each case is based on the total weight of the pure oil-based composition.

According to the use as mentioned above, the pure oil-based hair conditioner compositions preferably contain:
(a) 0.1-30 wt% of alkyl glycoside modified polysiloxanes;
(b) 30-85 wt% of volatile hydrophobic solvents;
(c) 5-30 wt% of non-volatile oils containing 0.5-30 wt% of dimethiconols;
(d) 0.5-10 wt% of dimethicone/vinyl dimethicone crosspolymer where the percentage in each case is based on the total weight of the pure oil-based composition.

According to the use as mentioned above, the pure oil-based personal care products are preferably skin care compositions, more preferably facial skin care compositions.

According to the use as mentioned above, the pure oil-based skin care compositions preferably contain:
(a) 0.1-10 wt% of alkyl glycoside modified polysiloxanes;
(b) 10-90 wt% of volatile hydrophobic solvents;
(c) 5-80 wt% of non-volatile oils containing 0.5-10 wt% of dimethiconols;
(d) 0.1-10 wt% of dimethicone/vinyl dimethicone crosspolymer where the percentage in each case is based on the total weight of the pure oil-based composition.

According to the use as mentioned above, the pure oil-based skin care compositions preferably contain:
(a) 0.1-10 wt% of alkyl glycoside modified polysiloxanes;
(b) 10-70 wt% of volatile hydrophobic solvents;
(c) 19-80 wt% of non-volatile oils containing 0.5-10 wt% of dimethiconols;
(d) 0-10 wt% of dimethicone/vinyl dimethicone crosspolymer
(e) 0.5-50 wt% of powders that can be used in the skin care compositions;
where the percentage in each case is based on the total weight of the pure oil-based composition.

According to the use as mentioned above, the pure oil-based skin care compositions preferably contain:
(a) 0.1-10 wt% of alkyl glycoside modified polysiloxanes;
(b) 10-70 wt% of volatile hydrophobic solvents;
(c) 19-75 wt% of non-volatile oils containing 0.5-10 wt% of dimethiconols;
(d) 0.1-10 wt% of dimethicone/vinyl dimethicone crosspolymer
(e) 10-50 wt% of powders that can be used in the skin care compositions;
where the percentage in each case is based on the total weight of the pure oil-based composition.

The components (a), optionally (b), optionally (c) and optionally (d) and optionally (e) are used in each case in such amounts that they add up to 100 wt %.

### Description of the Preferred Embodiments

The information on the products herein is as follows:
BELSIL® GB 1020 by Wacker Chemie AG, comprising 12 wt% of dimethiconols and 88 wt% of dimethicones, wherein the viscosity of dimethicones is 5 mPa.s (at 25°C according to DIN 53019) .
BELSIL® EG 2 by Wacker Chemie AG, comprising 14 wt% of dimethicone/vinyl dimethicone crosspolymers and 86 wt% of cyclopentasiloxane.
BELSIL® SPG 128 VP by Wacker Chemie AG, comprising 20 wt% of caprylyl dimethicone ethoxy glucoside and 80 wt% of cyclopentasiloxane.
BELSIL® WO 5000 by Wacker Chemie AG, comprising 30 wt% of caprylyl dimethicone ethoxy glucoside and 70 wt% of dimethicones, wherein the viscosity of dimethicone is 5 mPa.s (at 25°C according to DIN 53019) .
BELSIL® DM 5 by Wacker Chemie AG, comprising 100 wt% of dimethicones having a viscosity of 5 mPa.s (at 25°C according to DIN 53019).
BELSIL® CM 040 CN by Wacker Chemie AG, comprising 100 wt% of cyclopentasiloxane.
   Wacker Chemie AG's alkyl glycoside polysiloxane elastomer gel samples, whose polysiloxane molecular chains contain silicone resin segments, and which are prepared in reference to Sample 14 in Table 5 of WO2015091378A and contain alkyl glycoside polysiloxane elastomers.
KF-6104 by ShinEtsu, comprising polyglyceryl-3 polydimethylsiloxyethyl dimethicone.
BELSIL® PDM 20 by Wacker Chemie AG, comprising 100 wt% of trimethlysiloxyphenyl dimethicone
Landpowder Si BB88231 by Shanghai Kingland Fine Chemistry's Co., Ltd., a mixture of titanium dioxide and color pigment powders.

### Pure oil-based hair conditioner compositions

The following ingredients were mixed and stirred at a medium rotating speed of 200-300 rpm at room temperature to obtain Examples 1-3 (abbreviation Ex. 1-3) and Comparative Examples 1-3 (abbreviation C. Ex. 1-3).

**Table 1**

| Pure oil-based hair conditioner compositions | | Ex. 1 | C. Ex. 1 | Ex. 2 | C. Ex. 2 | C. Ex. 3 | Ex. 3 |
|---|---|---|---|---|---|---|---|
| INCI Names | Material from | wt% | wt% | wt% | wt% | wt% | wt% |
| Dimethicone | BELSIL® DM 5 | 70 | 70 | 15.0 | 15.0 | 23.0 | 15.0 |
| Polyglyceryl-3 polydimethylsiloxyethyl dimethicone | KF-6104 | / | 30.0 | / | / | / | / |
| Alkyl glycoside polysiloxane elastomer | Alkyl glycoside polysiloxane elastomer gel sample | / | / | / | / | 2.0 | / |
| Dimethiconol | BELSIL® GB 1020 | / | / | 1.2 | / | / | 1.2 |
| Cyclopentasiloxane | BELSIL® CM 040 CN | / | / | 81.8 | 83.0 | 75.0 | 79.0 |
| dimethicone/vinyl dimethicone crosspolymer | BELSIL® EG 2 | / | / | / | / | / | 2.8 |
| Caprylyl dimethicone ethoxy glucoside | BELSIL® SPG 128 VP | / | / | 2.0 | 2.0 | / | 2.0 |
| Caprylyl dimethicone ethoxy glucoside | BELSIL® WO 5000 | 30.0 | / | / | / | / | / |

Tested according to ISO7027 standard, each of the 3 compositions prepared in Examples 1-3 herein had a turbidity of less than 30NTU.

Naturally straight Chinese hair, never dyed or permed, was used for experimental purposes in the invention, and each hair tress measured 26 cm long and weighed 12 g.

1.4 ml of Pantene Pure Nourish (product number 98573610) Shampoo commercially available in 2015 was used to wash each straight hair tress, which was then air dried.

In Examples 1-3 and Comparative Examples 1-3, 0.7 ml of individual care products was evenly applied to the corresponding hair tress which has been cleaned and dried as above. The period of 10 seconds starting from applying the care products on the hair tress was defined as the early stage of application. The period following 60 seconds after the care products were applied on the hair tress was defined as the later stage of application.

A rating system is as follows:

| Performance rating | 1 | 2 | 3 | 4 | 5 |
|---|---|---|---|---|---|
| Hair smoothness in the early stage of application | Not smooth at all; typical effect with no care product being used; significant resistance when touching hair tresses following the growth direction of hair cuticles | Not very smooth | Average | Smooth in reference to the effect from using the same amount of L'ORÉAL Extraordinary Oil 2K060 | Very smooth |
| Hair stickiness in the later stage of application | Very sticky in reference to the effect from using the same amount of lanolin wax | Sticky | Average | Not very sticky in reference to the effect from using the same amount of L'ORÉAL Extraordinary Oil 2K060 | Not sticky |
| Hair fluffiness in the later stage of application | Not fluffy in reference to the effect from using the same amount of lanolin wax | Slightly fluffy | Average | Fluffy in reference to the effect from using the same amount of L'ORÉAL Extraordinary Oil 2K060 | Very fluffy |

Property values of the pure oil-based hair conditioner compositions of the examples and comparative examples are listed in Table 2.

**Table 2**

| | Ex. 1 | C. Ex. 1 | Ex. 2 | C. Ex. 2 | C. Ex. 3 | Ex. 3 |
|---|---|---|---|---|---|---|
| Hair smoothness in the early stage of application | 5 | 3 | 5 | 4 | 3 | 5 |
| Hair stickiness in the later stage of application | 5 | 3 | 5 | 5 | 5 | 5 |
| Hair fluffiness in the later stage of application | 1 | 1 | 1 | 1 | 2 | 4 |

The hair tress was not smooth after treated with the product prepared in Comparative Example 3. The reason for this is that the alkyl glycoside polysiloxane elastomer has nonlinear polysiloxane molecular chains, and the silicone segments cannot contribute to smoothness effect. In addition, the product obtained in Example 3 by the addition of dimethicone/vinyl dimethicone crosspolymer significantly increased the fluffiness of hair.

### Example 4

| Phase | Trade names of ingredients and substances contained | Suppliers | wt% |
|---|---|---|---|
| A | BELSIL® SPG 128 VP | Wacker Chemie | 10.0 |
| | containing 20 wt% of caprylyl | | |
| | dimethicone ethoxy glucoside | | |
| | BELSIL® GB 1020 | Wacker Chemie | 30.0 |
| | containing 12 wt% of dimethiconol | | |
| | BELSIL® PDM 20 | Wacker Chemie | 5.0 |
| | containing 100 wt% of | | |
| | trimethlysiloxyphenyl dimethicone | | |
| | IDD | DowPol | 50.0 |
| | containing 100 wt% of isododecane | | |
| | IPP | BASF | 5.0 |
| | containing 100 wt% of ethylhexyl palmitate | | |

In Example 4, the smoothness effect of the product was significantly improved by the addition of 3.6 wt% of dimethiconol, based on the total amount of composition.

### Pure oil-based skin/face care compositions

To prepare the skin massage oils of Example 5 and Comparative Example 5, oil phase ingredients were mixed one by one and stirred at a medium speed of 200-300 rpm at room temperature. To prepare the oily liquid foundations of Examples 6 and 7 and Comparative Examples 6 and 7, oil phase ingredients were mixed one by one and stirred well at a medium speed at room temperature. Afterwards, the powder was added to and stirred with the mixture of oils at a medium speed of 200-300 rpm for 2 minutes and then homogenized at 10,000 rpm for 3 minutes.

**Table 3**

| Pure oil-based skin care compositions | | Ex. 5 | C. Ex. 5 | Ex. 6 | C. Ex. 6 | C. Ex. 7 | Ex. 7 |
|---|---|---|---|---|---|---|---|
| INCI Names | Material from | wt% | wt% | wt% | wt% | wt% | wt% |
| Dimethicone | BELSIL® DM 5 | 10.0 | 10.0 | 10.0 | 10.0 | 18.0 | 10.0 |
| Polyglyceryl-3 polydimethylsiloxyethyl dimethicone | KF-6104 | / | 0.2 | / | 2.0 | / | / |
| Alkyl glycoside polysiloxane elastomer | Alkyl glycoside polysiloxane elastomer gel sample | / | / | / | / | 2.0 | / |
| Dimethiconol | BELSIL® GB 1020 | / | / | 0.5 | 0.5 | 0.5 | 0.5 |
| Cyclopentasiloxane | BELSIL® CM 040 CN | 89.8 | 89.8 | 72.5 | 72.5 | 6 4 . | 69.7 |
| 0-10 wt% of dimethicone/viny l dimethicone crosspolymer | BELSIL® EG 2 | / | / | / | / | / | 2.8 |
| Caprylyl dimethicone ethoxy glucoside | BELSIL® SPG 128 VP | 0.2 | / | 2.0 | / | / | 2.0 |
| Titanium dioxide powder | SiBB2231 | / | / | 15.0 | 15.0 | 15.0 | 15.0 |

The skin and face care compositions prepared in Examples 5-7 and Comparative Examples 5-7 herein were applied to the inside of the arm with gentle massage in circular motions using the middle finger until absorbed completely, and evaluated for their stickiness or smoothness 5 minutes later.

The stickiness (hand feel) was evaluated in reference to the following:
The stickiness of lanolin wax was given 10 points (very sticky);
Jergens Aloe and Lanolin (commercial product) oil-in-water lotion was given 5 points (moderately sticky);
The stickiness of baby oil (pure mineral oil) was given 0 point (not significantly sticky).

The smoothness was evaluated in reference to the following:
Talcum powder as a reference was given 10 points (significantly smooth);
Jergens Aloe and Lanolin (commercial product) was given 5 points (moderately smooth);
Water as a reference was given 0 point (not significantly smooth);

The compositions were evaluated for their storage stabilities, especially powder suspensibility, in reference to the following:
Fully suspended powder having no layering or phase separation after letting stand for 4 hours at room temperature was given 10 points;
Moderately suspended powder having slight layering without any clear interface after letting standing for 4 hours at room temperature was given 5 points;
Non-suspended powder having a clear layered interface within 1 hour after letting stand at room temperature was given 0 point.

Property values of the pure oil-based face-care compositions of examples and comparative examples are listed in Table 4. The experiments showed that polyglyceryl-3 polydimethylsiloxyethyl dimethicone and alkyl glycoside polysiloxane elastomer did not show the ability to help with powder suspensibility in Comparative Example 6 and 7. In Examples 6 and 7, 2 wt% of caprylyl dimethicone ethoxy glucoside was used to obtain a homogeneous and stable pure oil-based liquid foundation having no layering or phase separation and a power content up to 15 wt%.

**Table 4**

| | Ex. 5 | C. Ex. 5 | Ex. 6 | C. Ex. 6 | C. Ex. 7 | Ex. 7 |
|---|---|---|---|---|---|---|
| Stickiness | 6 | 7 | 5 | 6 | 5 | 5 |
| Smoothness | 7 | 6 | 7 | 5 | 7 | 8 |
| Powder suspensibility | / | / | 10 | 0 | 0 | 10 |

### Example 8: massage oil for skin care

| Phase | Trade names of ingredients and substances contained | suppliers | wt% |
|---|---|---|---|
| A | IDD | DowPol | 50.0 |
| | containing 100 wt% of isododecane | | |
| | BELSIL® GB 1020 | Wacker Chemie | 25.0 |
| | containing 12 wt% of dimethiconol | | |
| | BELSIL® SPG 128 VP | Wacker Chemie | 10.0 |
| | containing 20 wt% of caprylyl | | |
| | dimethicone ethoxy glucoside | | |
| | Olive oil | Lipo Chemicals | 5.00 |
| | Jojoba oil | Blue Star | 5.00 |
| | Macadamia oil | Lipo Chemicals | 5.00 |

The product prepared in Example 8 contained 3 wt% of dimethiconol and scored 8 points for its smoothness, which was significantly improved.

## Claims

1. Use of alkyl glycoside modified polysiloxanes in pure oil-based personal care products, wherein the pure oil-based products comprise less than or equal to 1 wt% of water, preferably less than or equal to 0.1 wt% of water, and the structure of the alkyl glycoside modified polysiloxanes is as shown in Formula (I) 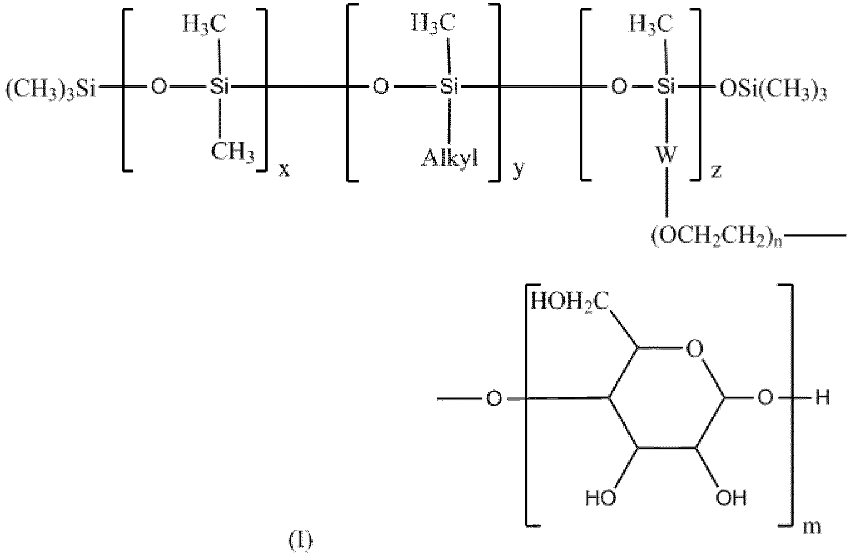 where
x, y and z are the same or different and respectively represent positive integers between 1 and 10,000, preferably between 1 and 5,000;
n and m are the same or different and respectively represent positive integers between 1 and 20, preferably between 1 and 15;
Alkyl represents a linear or branched C₂-C₂₀ hydrocarbon chain; preferably at least one alkyl group selected from a group of, ethyl, propyl, butyl, pentyl, hexyl, heptyl and octyl;
W represents a linear or branched C₂-C₂₀ alkylene selected from a group of ethylene, propylene, butylene, pentylene, hexylene, heptylene and octylene,
wherein the pure oil-based personal care products are pure oil-based hair conditioner compositions containing:
(a) 0.1-50 wt% of alkyl glycoside modified polysiloxanes;
(b) 0-85 wt% of volatile hydrophobic solvents;
(c) 0-20 wt% of non-volatile oils;
(d) 0.1-10 wt% of dimethicone/vinyl dimethicone crosspolymer
where the amount of components (b) and (c) cannot be zero at the same time and the percentage in each case is based on the total weight of the pure oil-based compositions.

2. Use according to Claim 1, wherein the pure oil-based personal care products are leave-on hair conditioner compositions having a turbidity of less than 80 NTU (**N**ephelometric **T**urbidity **U**nit) tested according to ISO 7027-1:2016 standard, preferably less than 30 NTU.

3. Use according to Claims 1 or 2, wherein the pure oil-based hair conditioner compositions is containing
(a) 0.1-30 wt% of alkyl glycoside modified polysiloxanes;
(b) 30-85 wt% of volatile hydrophobic solvents;
(c) 5-30 wt% of non-volatile oils containing 0.5-30 wt% of dimethiconols;
(d) 0.5-10 wt% of dimethicone/vinyl dimethicone crosspolymer;
where the percentage in each case is based on the total weight of the pure oil-based composition.

## Patentansprüche

1. Verwendung von alkylglucosidmodifizierten Polysiloxanen in reinölbasierten Körperpflegeprodukten, wobei die reinölbasierten Produkte kleiner oder gleich 1 Gew.-% Wasser, vorzugsweise kleiner oder gleich 0,1 Gew.-% Wasser, umfassen und die Struktur der alkylglucosidmodifizierten Polysiloxane wie in Formel (I) gezeigt ist: 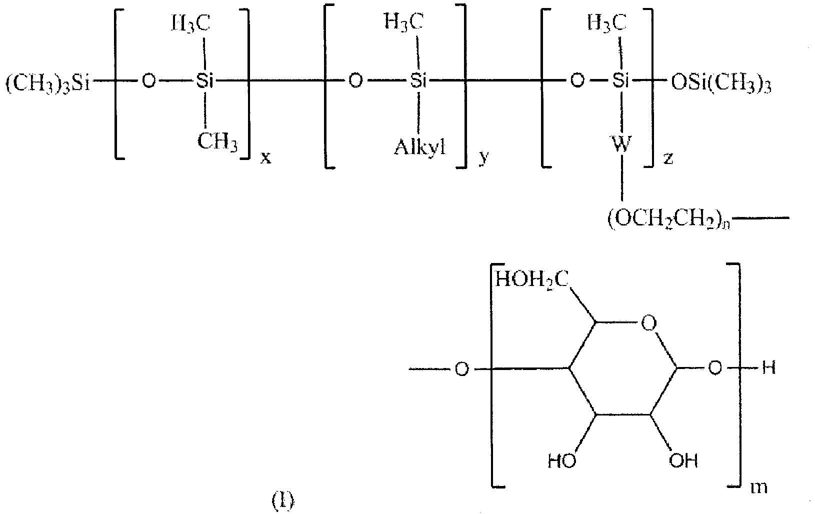 wobei
x, y und z gleich oder verschieden sind und jeweils für positive ganze Zahlen zwischen 1 und 10.000, vorzugsweise zwischen 1 und 5000, stehen;
n und m gleich oder verschieden sind und jeweils für positive ganze Zahlen zwischen 1 und 20, vorzugsweise zwischen 1 und 15, stehen;
Alkyl für eine lineare oder verzweigte C₂-C₂₀-Kohlenwasserstoffkette, vorzugsweise mindestens eine Alkylgruppe aus der Gruppe Ethyl, Propyl, Butyl, Pentyl, Hexyl, Heptyl und Octyl, steht;
W für ein lineares oder verzweigtes C₂-C₂₀-Alkylen aus der Gruppe Ethylen, Propylen, Butylen, Pentylen, Hexylen, Heptylen und Octylen steht; wobei es sich bei den reinölbasierten Körperpflegeprodukten um reinölbasierte Haarconditionerzusammensetzungen, die
(a) 0,1-50 Gew.-% alkylglucosidmodifizierte Polysiloxane;
(b) 0-85 Gew.-% flüchtige hydrophobe Lösungsmittel;
(c) 0-20 Gew.-% nichtflüchtige Öle;
(d) 0,1-10 Gew.-% Dimethicone/Vinyl Dimethicone Crosspolymer
enthalten, handelt, wobei die Menge der Komponenten (b) und (c) nicht gleichzeitig gleich null sein kann und die Prozentangabe jeweils auf dem Gesamtgewicht der reinölbasierten Zusammensetzungen basiert.

2. Verwendung nach Anspruch 1, wobei es sich bei den reinölbasierten Körperpflegeprodukten um Leave-On-Haarconditionerzusammensetzungen mit einer Trübheit von weniger als 80 NTU (**N**ephelometric **T**urbidity **U**nit) gemäß ISO-Norm 7027-1:2016, vorzugsweise weniger als 30 NTU, handelt.

3. Verwendung nach Anspruch 1 oder 2, wobei die reinölbasierten Haarconditionerzusammensetzungen
(a) 0,1-30 Gew.-% alkylglucosidmodifizierte Polysiloxane;
(b) 30-85 Gew.-% flüchtige hydrophobe Lösungsmittel;
(c) 5-30 Gew.-% nichtflüchtige Öle, die 0,5-30 Gew.-% Dimethiconole enthalten;
(d) 0,5-10 Gew.-% Dimethicone/Vinyl Dimethicone Crosspolymer
enthalten, wobei die Prozentangabe jeweils auf dem Gesamtgewicht der reinölbasierten Zusammensetzungen basiert.

## Revendications

1. Utilisation de polysiloxanes modifiés par des groupes alkyle et glycoside dans des produits de soins personnels à base d'huile pure, dans laquelle les produits à base d'huile pure comprennent une quantité inférieure ou égale à 1 % en poids d'eau, de préférence une quantité inférieure ou égale à 0,1 % en poids d'eau, et la structure des polysiloxanes modifiés par des groupes alkyle et glycoside est telle que représentée dans la formule (I) 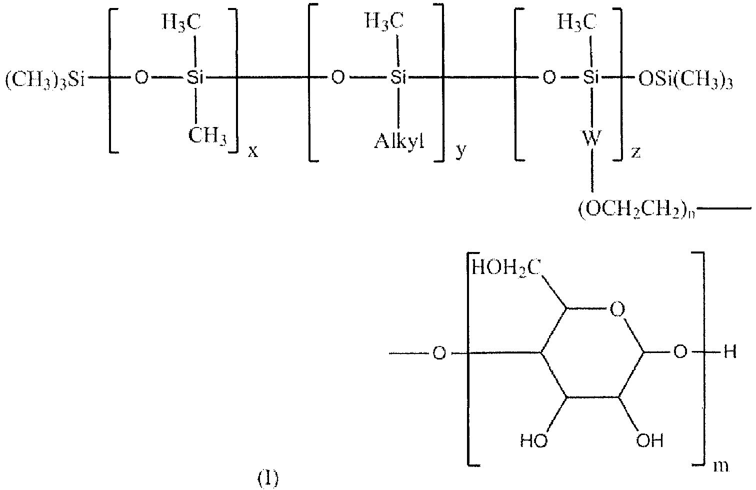 où
x, y et z sont identiques ou différents et représentent respectivement des nombres entiers positifs compris entre 1 et 10 000, de préférence entre 1 et 5 000 ;
n et m sont identiques ou différents et représentent respectivement des nombres entiers positifs compris entre 1 et 20, de préférence entre 1 et 15 ;
Alkyl représente une chaîne hydrocarbonée en C₂-C₂₀ linéaire ou ramifiée ; de préférence au moins un groupe alkyle choisi dans un groupe constitué par les groupes éthyle, propyle, butyle, pentyle, hexyle, heptyle et octyle ;
W représente un groupe alkylène en C₂-C₂₀ linéaire ou ramifié choisi dans un groupe constitué par les groupes éthylène, propylène, butylène, pentylène, hexylène, heptylène et octylène,
dans laquelle les produits de soins personnels à base d'huile pure sont des compositions d'après-shampooing à base d'huile pure contenant :
(a) 0,1-50 % en poids de polysiloxanes modifiés par des groupes alkyle et glycoside ;
(b) 0-85 % en poids de solvants hydrophobes volatils ;
(c) 0-20 % en poids d'huiles non volatiles ;
(d) 0,1-10 % en poids de polymère croisé de diméthicone/vinyldiméthicone,
la quantité des composants (b) et (c) ne pouvant pas être nulle en même temps et le pourcentage dans chaque cas étant par rapport au poids total des compositions à base d'huile pure.

2. Utilisation selon la revendication 1, dans laquelle les produits de soins personnels à base d'huile pure sont des compositions d'après-shampooing sans rinçage ayant une turbidité mesurée selon la norme ISO 7027-1:2016 inférieure à 80 uTN (**u**nités de **t**urbidité **n**éphélémétrique) de préférence inférieure à 30 uTN.

3. Utilisation selon les revendications 1 ou 2, dans laquelle les compositions d'après-shampooing à base d'huile pure contiennent
(a) 0,1-30 % en poids de polysiloxanes modifiés par des groupes alkyle et glycoside ;
(b) 30-85 % en poids de solvants hydrophobes volatils ;
(c) 5-30 % en poids d'huiles non volatiles contenant 0,5-30 % en poids de diméthiconols ;
(d) 0,5-10 % en poids de polymère croisé de diméthicone/vinyldiméthicone,
le pourcentage dans chaque cas étant par rapport au poids total de la composition à base d'huile pure.
